(19)

Europäisches
Patentamt

European
Patent Office

Office européen
des brevets

(11) **EP 4 710 848 A1**

(12) **EUROPEAN PATENT APPLICATION**

(43) Date of publication:
**18.03.2026 Bulletin 2026/12**

(21) Application number: **25201668.8**

(22) Date of filing: **11.09.2025**

(51) International Patent Classification (IPC):
***A61B 5/024*** *(2006.01)* ***A61B 5/00*** *(2006.01)*

(52) Cooperative Patent Classification (CPC):
**A61B 5/02416; A61B 5/7221**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **12.09.2024 CN 202411281878**

(71) Applicant: **Shenzhen Goodix Technology Co., Ltd.
Shenzhen, Guangdong 518045 (CN)**

(72) Inventor: **WEN, Yongfeng
Shenzhen, 518045 (CN)**

(74) Representative: **Kraus & Lederer PartGmbB
Thomas-Wimmer-Ring 15
80539 München (DE)**

(54) **DATA PROCESSING METHOD, ELECTRONIC DEVICE, AND STORAGE MEDIUM**

(57) A data processing method, an electronic device, and a storage medium are provided. The method is applied to the processing module, and includes: processing an analog signal obtained by sampling a to-be-measured signal based on a target parameter to obtain a first digital quantity, wherein the target parameter is used to indicate a signal value of a signal for cancelling at least a portion of the analog signal; compensating the first digital quantity based on a target compensation amount to obtain a measured signal value of the analog signal; updating, when the first digital quantity satisfies a parameter update condition, the target parameter based on the first digital quantity to obtain an updated target parameter; and updating the target compensation amount based on the updated target parameter to obtain an updated target compensation amount.

FIG. 2

**Description**

TECHNICAL FIELD

Embodiments of the present disclosure relate to the technical field of data processing, and particularly relate to a method for data processing, an electronic device, and a storage medium.

BACKGROUND

At present, during signal acquisition of an analog signal, the analog signal is usually converted into a digital signal for acquisition based on factors such as the quality and costs of an acquired signal.

For example, during acquisition of a photoplethysmography (PPG) signal, a light-emitting element can emit light to irradiate skin surface. A receiving element receives a reflected light signal, converts the light signal into the PPG signal, and then inputs the PPG signal into a chip. The chip converts the PPG signal into a corresponding digital signal, outputs the digital signal, and then computes a measured value of the PPG signal based on the digital signal, thereby acquiring the PPG signal.

However, a signal value of the PPG signal is in a wide range, and processable signal values for the chip are in a limited range. Therefore, the chip often fails to acquire valid data during acquisition of the PPG signal because the PPG signal is either too large or too small, thereby resulting in low signal acquisition efficiency.

SUMMARY

In view of this, embodiments of the present disclosure provide a method for data processing, a processing module, an electronic device, and a storage medium, to at least partially solve the above problems.

According to an embodiment in a first aspect of the present disclosure, a method for data processing is provided, wherein the method is applied to a processing module, and comprises: processing an analog signal obtained by sampling a to-be-measured signal based on a target parameter to obtain a first digital quantity, wherein the target parameter is used to indicate a signal value of a signal for cancelling at least a portion of the analog signal; compensating the first digital quantity based on a target compensation amount to obtain a measured signal value of the analog signal; updating, when the first digital quantity satisfies a parameter update condition, the target parameter based on the first digital quantity to obtain an updated target parameter; and updating the target compensation amount based on the updated target parameter to obtain an updated target compensation amount.

According to an embodiment in a second aspect of the present disclosure, a processing module is provided, comprising: a signal processing unit configured to process an analog signal obtained by sampling a to-be-measured signal based on a target parameter to obtain a first digital quantity, wherein the target parameter is used to indicate a signal value of a signal for cancelling at least a portion of the analog signal; a value compensation unit configured to compensate the first digital quantity based on a target compensation amount to obtain a measured signal value of the analog signal; a first update unit configured to update, when the first digital quantity satisfies a parameter update condition, the target parameter based on the first digital quantity to obtain an updated target parameter; and a second update unit configured to update the target compensation amount based on the updated target parameter to obtain an updated target compensation amount.

According to an embodiment in a third aspect of the present disclosure, an electronic device is provided, comprising: a processor, a memory, a communications interface, and a communication bus, wherein the processor, the memory, and the communications interface are configured to complete communication with each other through the communication bus; and the memory is configured to store at least one executable instruction, wherein the executable instruction causes the processor to implement corresponding operations of the method in the above first aspect.

According to an embodiment in a fourth aspect of the present disclosure, a computer storage medium is provided, storing a computer program thereon, wherein the program, when executed by a processor, implements the method in the above first aspect.

According to an embodiment in a sixth aspect of the present disclosure, a computer program product is provided, comprising a computer instruction, wherein the computer instruction instructs a computing device to implement the method in the above first aspect.

According to the data processing solutions provided in the embodiments of the present disclosure, an analog signal obtained by sampling a to-be-measured signal is processed based on a target parameter to obtain a first digital quantity, then the first digital quantity is compensated based on a target compensation amount to obtain a measured signal value of the analog signal, the target parameter is updated based on the first digital quantity when the first digital quantity satisfies a parameter update condition, to obtain an updated target parameter, and the target compensation amount is updated based on the updated target parameter to obtain an updated target compensation amount. Therefore, during processing of the analog signal obtained by sampling the to-be-measured signal based on the target parameter, at least a portion of the analog signal is canceled with a signal of a signal value indicated by the target parameter, so that the first digital quantity is determined based on the cancellation signal, and then the first digital signal is compensated based on the target compensation amount, to obtain the measured signal value of the analog signal, and acquire the to-be-measured signal. The target parameter and the target compensation amount can be updated based on the first digital quantity. Compared with determining the first digital quantity directly based on the analog signal, the processable signal range for the processing module is extended by setting the target parameter and the target compensation amount in the present disclosure, thereby minimizing the problem that the processing module fails to acquire valid data during acquisition of the to-be-measured signal because the to-be-measured signal is either too large or too small, improving the efficiency of the measured signal value of the analog signal obtained by acquisition of the to-be-measured signal, and then improving the efficiency of signal acquisition.

BRIEF DESCRIPTION OF DRAWINGS

To more clearly describe technical solutions of embodiments of the present disclosure or the prior art, drawings to be used in the description of the embodiments or the prior art will be briefly introduced below. Apparently, the drawings in the description below are merely some embodiments disclosed in the embodiments of the present disclosure. For those of ordinary skills in the art, other drawings may also be obtained based on these drawings.

FIG. 1 is a flowchart of a method for data processing in an embodiment of the present disclosure;

FIG. 2 is a schematic diagram of a measured signal value in an embodiment of the present disclosure;

FIG. 3 is a schematic diagram of a processing module in an embodiment of the present disclosure; and

FIG. 4 is a schematic diagram of an electronic device in an embodiment of the present disclosure.

DETAILED DESCRIPTION

Method for Data Processing

An embodiment of the present disclosure provides a method for data processing, the method for data processing is applied to a processing module, the processing module may be, e.g., a chip for acquiring a PPG signal, which is not limited in the embodiments of the present disclosure.

The method for data processing is detailed below with reference to a plurality of embodiments.

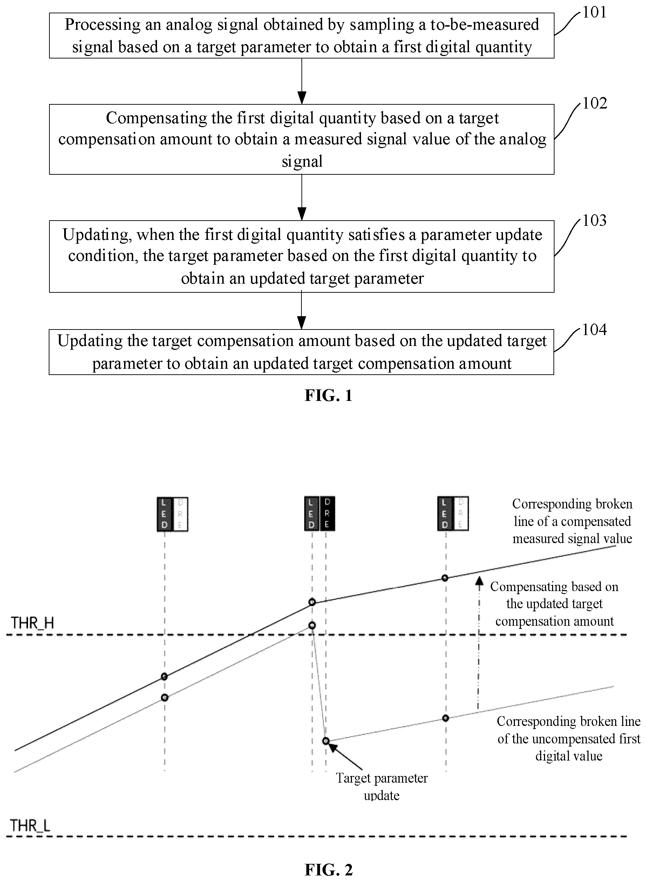

FIG. 1 is a flowchart of a method for data processing in an embodiment of the present disclosure. As shown in FIG. 1, the method for data processing includes the following steps:

Step 101: processing an analog signal obtained by sampling a to-be-measured signal based on a target parameter to obtain a first digital quantity.

The target parameter is used to indicate a signal value of a signal for cancelling at least a portion of the analog signal.

In a specific embodiment, the to-be-processed signal is a PPG signal. The processing module samples the PPG signal to obtain the analog signal. Then, the processing module cancels at least a portion of the analog signal based on the target parameter stored in a parameter register within the processing module, and then processes the cancellation signal to obtain the first digital quantity, wherein an initial value of the target parameter may be 0, or may not be 0, which is not limited in the embodiments of the present disclosure.

Based on the analog signal being a signal obtained by sampling the PPG, at least a portion of the above canceled analog signal may be at least a portion of direct traffic of the analog signal, the signal that cancels the at least a portion of the analog signal is a current signal in an opposite direction to the direct traffic of the analog signal, and the signal value is a signal value indicated by the target parameter. It should be noted that the signal value indicated by the target parameter is generally less than the direct traffic of the analog signal, so that basically the analog signal will not be entirely canceled, nor will a reversed analog signal be obtained.

Step 102: compensating the first digital quantity based on a target compensation amount to obtain a measured signal value of the analog signal.

When the initial value of the target parameter is 0, an initial value of the target compensation amount is also 0. When the initial value of the target parameter is not 0, the initial value of the target compensation amount is determined based on the target parameter.

In a specific embodiment, after obtaining the first digital quantity, the processing module determines a sum of the target compensation amount and the first digital quantity as a digital signal corresponding to the analog signal. The digital signal is the measured signal value of the analog signal, thus acquiring the to-be-measured signal. The measured signal value of the analog signal may be stored in a FIFO queue included in the processing module.

Step 103: updating, when the first digital quantity satisfies a parameter update condition, the target parameter based on the first digital quantity to obtain an updated target parameter.

The parameter update condition is used to indicate a condition that the processing module fails to acquire valid data because the target parameter is either too large or too small.

For example, when the first digital quantity is greater than or equal to a first threshold, or when the first digital quantity is less than or equal to a second threshold, the target parameter is updated based on the first digital quantity, to allow the first digital quantity subsequently obtained based on the updated target parameter to be between the first threshold and the second threshold as much as possible, wherein the second threshold is greater than the first threshold, and the first threshold and the second threshold generally may be set based on actual requirements, for example, the first threshold is -8388608 and the second threshold is 8388607, which is not limited in the embodiments of the present disclosure.

Step 104: updating the target compensation amount based on the updated target parameter to obtain an updated target compensation amount.

In an embodiment of the present disclosure, the analog signal obtained by sampling the to-be-measured signal is processed based on the target parameter to obtain the first digital quantity, then the first digital quantity is compensated based on the target compensation amount to obtain the measured signal value of the analog signal, the target parameter is updated based on the first digital quantity in response to the first digital quantity satisfying the parameter update condition, to obtain the updated target parameter, and the target compensation amount is updated based on the updated target parameter to obtain the updated target compensation amount. Therefore, during processing of the analog signal obtained by sampling the to-be-measured signal based on the target parameter, at least a portion of the analog signal is canceled with a signal of a signal value indicated by the target parameter, so that the first digital quantity is determined based on the cancellation signal, and then the first digital signal is compensated based on the target compensation amount, to obtain the measured signal value of the analog signal, and acquire the to-be-measured signal. The target parameter and the target compensation amount can be updated based on the first digital quantity. Compared with determining the first digital quantity directly based on the analog signal, the processable signal range for the processing module is extended by setting the target parameter and the target compensation amount in the present disclosure, thereby minimizing the problem that the processing module fails to acquire valid data during acquisition of the to-be-measured signal because the to-be-measured signal is either too large or too small, improving the efficiency of the measured signal value of the analog signal obtained by acquisition of the to-be-measured signal, and then improving the efficiency of signal acquisition.

In a possible implementation, the above analog signal is a current signal. For example, the to-be-measured signal is a PPG signal, and the analog signal is a current signal. On this basis, the above step 101 includes the following specific processing: outputting, via a signal output unit, a signal with a signal value indicated by the target parameter for cancelling the at least a portion of the analog signal to obtain a canceled analog signal; converting the canceled analog signal via a trans-impedance amplifier included in the processing module into a voltage signal; and converting the voltage signal via an analog-to-digital converter included in the processing module into the first digital quantity.

In an embodiment of the present disclosure, when the first digital quantity is obtained, after the at least a portion of the analog signal which is a current signal is canceled , the canceled analog signal can be converted into the voltage signal, and then the voltage signal can be converted into a digital quantity. Therefore, the analog signal can be converted into the digital quantity via the signal output unit, the trans-impedance amplifier, and the analog-to-digital converter, thus reducing the costs.

In a possible implementation, the processing module is configured to periodically sample the to-be-measured signal. On this basis, the method for data processing further includes the following specific processing:

determining that the first digital quantity satisfies the parameter update condition in response to the first digital quantity being a digital quantity corresponding to an analog signal obtained by an i-th sampling of the to-be-measured signal and a digital quantity corresponding to an analog signal obtained by each sampling among an (i-cnt+1)-th sampling to the i-th sampling of the to-be-measured signal being greater than or equal to a first threshold or being less than or equal to a second threshold, and otherwise determining that the first digital quantity fails to satisfy the parameter update condition, wherein each of the i and the cnt is a positive integer, the cnt is less than or equal to i, the first threshold is greater than the second threshold, and the cnt is, e.g., 1, 2, 4, or 8, which is not limited in the embodiments of the present disclosure.

In an example, when the cnt=1, and the first digital quantity is greater than or equal to the first threshold or is less than or equal to the second threshold, it is determined that the first digital quantity satisfies the parameter update conditions, while when the cnt>1, and a digital quantity corresponding to an analog signal obtained by each sampling among cnt continuous sampling of the to-be-measured signal is greater than or equal to the first threshold or is less than or equal to the second threshold, it is determined that the first digital quantity satisfies the parameter update condition, wherein the digital quantity corresponding to the analog signal obtained by a last sampling among the continuous cnt sampling is the first digital quantity.

In an embodiment of the present disclosure, when the cnt=1, and when the first digital quantity is greater than or equal to the first threshold or is less than or equal to the second threshold, it is determined that the first digital quantity satisfies the parameter update condition, so that the process of determining whether the first digital quantity satisfies the parameter update condition is simple and saves computing resources; while when the cnt>1, and when the digital quantity corresponding to the analog signal obtained by each sampling among the cnt continuous sampling of the to-be-measured signal is greater than or equal to the first threshold or is less than or equal to the second threshold, it is determined that the first digital quantity satisfies the parameter update condition. In this case, it is equivalent to additionally providing an anti-shake mechanism, improving the stability of the processing module, and further making the result of determining whether the first digital quantity satisfies the parameter update condition more accurate.

In a possible implementation, the "updating the target parameter based on the first digital quantity to obtain the updated target parameter" in the step 103 includes the following specific processing: computing the updated target parameter using a formula of:

dc_cancel_code1 = H3((Idc_cancel0 + PpgMixData * A * 1000000/(TIA * D)) * B/C + dc_cancel_code0);

wherein H3() is a third anti-overflow function, dc_cancel_code1 is the updated target parameter, Idc_cancel0 is a signal value indicated by the target parameter, Idc_cancel0 is in a unit of microampere, PpgMixData is the first digital quantity, A is a scale factor of the analog-to-digital converter, for example, 1.8, TIA is a gain resistance value of the trans-impedance amplifier, the TIA may be determined based on the number of gain resistors included in the trans-impedance amplifier, for example, when the trans-impedance amplifier comprises two gain resistors each with a resistance value of tia, the TIA=2*tia, the TIA is in a unit of ohm, D is a maximum value outputted from the analog-to-digital converter, for example, D=8388608, B is a maximum storage value at a storage bit used to store the target parameter, that is, a maximum value that can be stored at the storage bit, for example, 255 or 256, etc., C is a maximum signal value of an output signal from the signal output unit, for example, 32, 64, 128, or 256 microamperes, etc., and dc_cancel_code0 is the target parameter.

It should be noted that

H3((Idc_cancel0+PpgMixData*A*1000000/(TIA*D))*B/C+dc_cancel_code0) is used to represent that when (Idc_cancel0+PpgMixData*A*1000000/(TIA*D))*B/C+dc_cancel_code0 is less than or equal to a third threshold, H3((Idc_cancel0+PpgMixData*A*1000000/(TIA*D))*B/C+dc_cancel_code0) is equal to the third threshold; when (Idc_cancel0+PpgMixData*A*1000000/(TIA*D))*B/C+dc_cancel_code0 is greater than or equal to a fourth threshold, H3((Idc_cancel0+PpgMixData*A*1000000/(TIA*D))*B/C+dc_cancel_code0) is equal to the fourth threshold; and when (Idc_cancel0+PpgMixData*A*1000000/(TIA*D))*B/C+dc_cancel_code0 is greater than or equal to the third threshold and less than the fourth threshold, H3((Idc_cancel0+PpgMixData*A*1000000/(TIA*D))*B/C+dc_cancel_codeo) is equal to (Idc_cancel0+PpgMixData*A* 1000000/(TIA*D))*B/C+dc_cancel_code0; wherein the fourth threshold is greater than the third threshold, specific values of the third threshold and the fourth threshold may be a minimum value and a maximum value that can be stored at a storage bit used to store dc_cancel_code1 respectively. For example, the third threshold is 0, and the fourth threshold is 255.

In a specific embodiment, a signal value Idc_cancel1=Idc_cancel0+PpgMixData*A*1000000/(TIA*D) indicated by the updated target parameter may be first computed, and then the updated target parameter dc_cancel_code1=H3(Idc_cancel1*B/C+dc_cancel_code0) may be computed. In this case, Idc_cancel1 has practical physical meaning, thereby facilitating troubleshooting when the processing module is abnormal.

In another specific embodiment, an amplified signal value

Idc_cancel2=(Idc_cancel0+PpgMixData*A*1000000/(TIA*D))*B may be first computed, and then the updated target parameter dc_cancel_codel=H3(Idc_cancel2/C+dc_cancel_code0) may be computed. During computation of dc_cancel_code1, multiplication is no longer required, thereby improving the computing efficiency of the updated target parameter.

In an embodiment of the present disclosure, the updated target parameter can be determined easily and quickly with a computing formula of the updated target parameter dc_cancel_code 1.

In the step 104, the updated target compensation amount can be determined by at least two implementations below:

In a possible implementation, the step 104 includes the following specific processing: determining the updated target compensation amount based on a signal value indicated by the updated target parameter, a gain resistance value of the trans-impedance amplifier, and an analog-to-digital conversion rule of the analog-to-digital converter.

For example, a product of the signal value indicated by the updated target parameter and the gain resistance value is determined as a voltage value, then a digital quantity corresponding to the voltage value is determined based on the analog-to-digital conversion rule of the analog-to-digital converter, and the digital quantity is used as the updated target compensation amount.

In an embodiment of the present disclosure, it is convenient and efficient to determine the updated target compensation amount through theoretical deduction.

In another possible implementation, the step 104 includes the following specific processing: processing the analog signal obtained by sampling the to-be-measured signal based on the updated target parameter to obtain a second digital quantity; and updating the target compensation amount based on a difference between the first digital quantity and the second digital quantity to obtain the updated target compensation amount.

In a specific embodiment, after the updated target parameter is obtained, a sampling channel can be started by sampling the to-be-measured signal once to obtain the analog signal, and processing the analog signal based on the updated target parameter to obtain the second digital quantity. In this case, since the difference between the first digital quantity and the second digital quantity reflects a difference between the updated target parameter and the target parameter, the target compensation amount can be updated based on the difference between the first digital quantity and the second digital quantity, to obtain the updated target compensation amount.

In an embodiment of the present disclosure, compared with determining the updated target compensation amount through theoretical deduction based on the signal value indicated by the updated target parameter, the gain resistance value of the trans-impedance amplifier, and the analog-to-digital conversion rule of the analog-to-digital converter, in an embodiment of the present disclosure, the to-be-measured signal is re-sampled after updating the target parameter, and a current sampled analog signal is processed based on the updated target parameter to obtain the second digital quantity, without the need to calibrate the data such as the signal value indicated by the updated target parameter and the gain resistance value of the trans-impedance amplifier, and the determined updated target compensation amount is more accurate.

In a possible implementation, the updating the target compensation amount based on the difference between the first digital quantity and the second digital quantity to obtain the updated target compensation amount comprises: computing the updated target compensation amount using a formula of:

$$dre\_code1 = H1(Rightshift(PpgMixData - PpgDreData, scale) + dre\_code0);$$

wherein dre_code1 is the updated target compensation amount, H1() is a first anti-overflow function, Rightshift(PpgMixData-PpgDreData, scale) is a value obtained by right shifting a difference between PpgMixData and PpgDreData by scale bits (rounding off may be performed during the right shifting), PpgMixData is the first digital quantity in a binary form, PpgDreData is the second digital quantity in a binary form, scale is the number of right shifted bits, the scale is generally any integer of 1-8, optionally, the scale may be any integer of 1-6, and dre_code0 is the target compensation amount.

It should be noted that H1(Rightshift(PpgMixData-PpgDreData,scale)+dre_code0) is used to represent that when Rightshift(PpgMixData-PpgDreData,scale)+dre_code0 is less than or equal to a fifth threshold, H1(Rightshift(PpgMixData-PpgDreData,scale)+dre_code0) is equal to the fifth threshold; when Rightshift(PpgMixData-PpgDreData,scale)+dre_code0 is greater than or equal to a sixth threshold, H1(Rightshift(PpgMixData-PpgDreData,scale)+dre_code0) is equal to the sixth threshold; and when Rightshift(PpgMixData-PpgDreData,scale)+dre_code0 is greater than or equal to the fifth threshold and less than or equal to the sixth threshold, H1(Rightshift(PpgMixData-PpgDreData,scale)+dre_code0) is equal to Rightshift(PpgMixData-PpgDreData,scale)+dre_code0; wherein specific values of the fifth threshold and the sixth threshold may be a minimum value and a maximum value that can be stored at a storage bit used to store dre_code1 respectively. For example, the fifth threshold is -8388608, and the sixth threshold is 8388607.

On this basis, the step 102 includes the following specific processing: computing the measured signal value of the analog signal using a formula of:

$$PPG = H2(Rightshift(PpgMixData, scale) + dre\_code0);$$

wherein PPG is the measured signal value of the analog signal, H2() is a second anti-overflow function, and Rightshift(PpgMixData, scale) is a value obtained by right shifting PpgMixData by scale bits (rounding off may be performed during the right shifting).

It should be noted that H2(Rightshift(PpgMixData, scale)+dre_code0) is used to represent that when Rightshift(PpgMixData, scale)+dre_code0 is less than or equal to a seventh threshold, H2(Rightshift(PpgMixData, scale)+dre_code0) is equal to the seventh threshold; when Rightshift(PpgMixData, scale)+dre_code0 is greater than or equal to an eighth threshold, H2(Rightshift(PpgMixData, scale)+dre_code0) is equal to the eighth threshold; and when Rightshift(PpgMixData-PpgDreData,scale)+dre_code0 is greater than or equal to the seventh threshold and is less than or equal to the eighth threshold, H2(Rightshift(PpgMixData, scale)+dre_code0) is equal to Rightshift(PpgMixData, scale)+dre_code0, wherein the eighth threshold is greater than the seventh threshold, and specific values of the seventh threshold and the eighth threshold may be a minimum value and a maximum value that can be stored at a storage bit used to store the PPG respectively. For example, the seventh threshold is -8388608, and the eighth threshold is 8388607.

In an embodiment of the present disclosure, the difference between the first digital quantity and the second digital quantity can be amplified by right shifting the difference between the first digital quantity and the second digital quantity, and then the updated target compensation amount can be amplified. Similarly, the measured signal value of the analog signal can be amplified by right shifting the first digital quantity. Therefore, when the measured signal is acquired, the measured signal value of the acquired analog signal can be amplified by 2-64 times, thereby improving the quality of the acquired data.

In a possible implementation, the processing module is configured to periodically sample the to-be-measured signal. On this basis, the data acquisition method further includes the following processing: stopping obtaining the measured signal value of the analog signal, in response to the first digital quantity being less than or equal to a lowest saturation threshold or being greater than or equal to a highest saturation threshold after obtaining the first digital quantity (that is, stopping performing the above steps 102, 103 and 104), until obtaining a first digital quantity less than the highest saturation threshold and greater than the lowest saturation threshold, and then performing the above steps 102, 103, and 104 based on the first digital quantity.

The lowest saturation threshold and the highest saturation threshold are determined based on a signal value range of processable signals for the processing module, primarily by a signal processing capacity of the trans-impedance amplifier and a signal processing capacity of the analog-to-digital converter. The first threshold is greater than the lowest saturation threshold, and the second threshold is less than the highest saturation threshold.

In a specific embodiment, the processing module comprises a range extension unit, at least the above step 102 is executed by the range extension unit, and optionally, the above steps 103 and 104 may also be executed by the range extension unit, which is not limited in the embodiments of the present disclosure. On this basis, when the measured signal is suddenly saturated during use of the range extension unit, such that the first digital quantity is less than or equal to the lowest saturation threshold or is greater than or equal to the highest saturation threshold. In this case, the value of the cnt in the range extension unit may be cleared to prevent the range extension unit from performing any further operations. The range extension unit can resume operations only after the current value of the sampled analog signal decreases, i.e., after the measured signal is no longer saturated.

In an embodiment of the present disclosure, when the first digital quantity is less than or equal to the lowest saturation threshold or is greater than or equal to the highest saturation threshold, since the corresponding analog signal is in a saturated state, the first digital quantity has a large error. In this case, processing is not continued based on the first digital quantity, thereby improving the accuracy of acquiring the to-be-measured signal.

In a possible implementation, the above step 102 is implemented by the range extension unit included in the processing module. On this basis, the data acquisition method further includes: resetting the range extension unit when the range extension unit is abnormal.

In a specific embodiment, when the range extension unit is abnormal in a particular scenario, a reset mechanism is required to restore the function of the range extension unit to an initial state. The range extension unit may be reset via a main switch of the processing module by clearing, e.g., the cnt, dre_code1, dre_code0, dc_cancel_code1, and dc_cancel_code0 in the range extension unit. When the main switch of the processing module is switched off, the range extension unit is reset. The range extension unit may also be reset via a reset switch of the range extension unit. When the reset switch of the range extension unit=1, the range extension unit is reset. In this case, if sampling has been started, resetting is required at the beginning of a next sampling, and the reset switch of the range extension unit will be automatically reset to 0 after 1 is written.

Thus, the range extension unit can be reset when the range extension unit is abnormal.

Optionally, after obtaining the updated target parameter, the range extension unit can be notified by an update flag (such as a DcCancelUpdateFlag) to be ready for starting a next round of work, that is, performing data compensation and other processing on a next first digital quantity.

Optionally, the first digital quantity and the second digital quantity obtained by the processing module may be results of ambient light suppression of the to-be-measured signal. The embodiments of the present disclosure do not impose any limitation on whether the processing module performs ambient light suppression on the to-be-measured signal. When the range extension unit compensates the first digital quantity, the situation where the ambient light changes automatically is minimized.

FIG. 2 is a schematic diagram of a measured signal value in an embodiment of the present disclosure. As shown in FIG. 2, based on the to-be-measured signal being a PPG signal, a LED is a light emitting device configured to emit light to irradiate skin surface and then generate a PPG signal. A block marked with LED is filled to indicate that the LED is emitting light, DRE is a range extension unit, a block marked with DRE is filled to indicate that the DRE is working, a block marked with DRE is not filled to indicate that the DRE is not working, THR_H is the second threshold, and THR_L is the first threshold. As can be seen from FIG. 2, after the target parameter is updated, the next first digital quantity can be processed based on the updated target parameter and the updated target compensation amount. Specifically, after the target parameter is updated, the processing module can process the analog signal obtained by the next sampling of the to-be-measured signal based on the updated target parameter to obtain a first digital quantity and then compensate the first digital quantity based on the updated target compensation amount to obtain the measured signal value of the analog signal.

Processing module

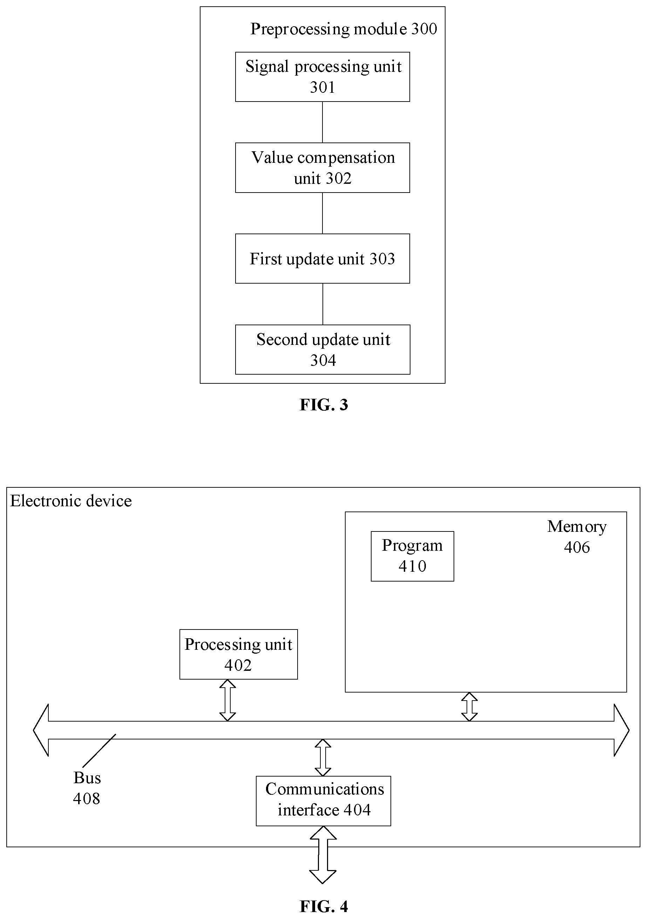

Corresponding to the above method embodiments, FIG. 3 shows a schematic diagram of a processing module in an embodiment of the present disclosure. As shown in FIG. 3, the processing module 300 comprises:

a signal processing unit 301 configured to process an analog signal obtained by sampling a to-be-measured signal based on a target parameter to obtain a first digital quantity, wherein the target parameter is used to indicate a signal value of a signal for cancelling at least a portion of the analog signal;

a value compensation unit 302 configured to compensate the first digital quantity based on a target compensation amount to obtain a measured signal value of the analog signal;

a first update unit 303 configured to update, when the first digital quantity satisfies a parameter update condition, the target parameter based on the first digital quantity to obtain an updated target parameter; and

a second update unit 304 configured to update the target compensation amount based on the updated target parameter to obtain an updated target compensation amount.

In an embodiment of the present disclosure, the signal processing unit 301 can process the analog signal obtained by sampling the to-be-measured signal based on the target parameter to obtain the first digital quantity, then the value compensation unit 302 can compensate the first digital quantity based on the target compensation amount to obtain the measured signal value of the analog signal, the first update unit 303 updates the target parameter based on the first digital quantity in response to the first digital quantity satisfying the parameter update condition, to obtain the updated target parameter, and the second update unit 304 updates the target compensation amount based on the updated target parameter to obtain the updated target compensation amount. Therefore, during processing of the analog signal obtained by sampling the to-be-measured signal based on the target parameter, at least a portion of the analog signal is canceled with a signal of a signal value indicated by the target parameter, so that the first digital quantity is determined based on the cancellation signal, and then the first digital signal is compensated based on the target compensation amount, to obtain the measured signal value of the analog signal, and acquire the to-be-measured signal. The target parameter and the target compensation amount can be updated based on the first digital quantity. Compared with determining the first digital quantity directly based on the analog signal, the processable signal range for the processing module is extended by setting the target parameter and the target compensation amount in the present disclosure, thereby minimizing the problem that the processing module fails to acquire valid data during acquisition of the to-be-measured signal because the to-be-measured signal is either too large or too small, improving the efficiency of the measured signal value of the analog signal obtained by acquisition of the to-be-measured signal, and then improving the efficiency of signal acquisition.

It should be noted that the processing module in this embodiment is configured to implement the corresponding method for data processing in the above method embodiments and has the beneficial effects of the corresponding method embodiments, which will not be repeated here.

Electronic device

FIG. 4 is a schematic block diagram of an electronic device provided in an embodiment of the present disclosure. Specific embodiments of the present disclosure do not impose any limitation on specific implementations of the electronic device. As shown in FIG. 4, the electronic device may comprise: a processor 402, a communications interface 404, a memory 406, and a communication bus 408, wherein:

the processor 402, the communications interface 404, and the memory 406 are configured to complete communication with each other through the communication bus 408.

The communications interface 404 is configured to communicate with other electronic devices or servers.

The processor 402 is configured to execute a program 410 and specifically may implement relevant steps of the method for data processing in any one of the above embodiments.

Specifically, the program 410 may comprise a program code. The program code comprises a computer operation instruction.

The processor 402 may be a CPU, or an Application Specific Integrated Circuit (ASIC), or one or more integrated circuits configured to implement embodiments of the present disclosure. One or more processors included in a smart device may be processors of a same type, e.g., one or more CPUs; or may be processors of different types, e.g., one or more CPUs and one or more ASICs.

RISC-V is an open-source instruction set architecture based on a reduced instruction set (RISC) principle, can be applied to various aspects such as a single chip microcomputer and a FPGA chip, and can be specifically applied to the fields, such as security of Internet of Things, industrial control, a mobile phone, and a personal computer. Moreover, it is designed based on actual situations such as a small size, a fast speed, and low power consumption, so that it is particularly adapted to a modern computing device, such as a warehouse-scale cloud computer, a high-end mobile phone, and a tiny embedded system. With the emergence of the artificial intelligence Internet of Things (AIoT), the RISC-V instruction set architecture has also attracted more and more attention and support, and is expected to become the next generation of a widely used CPU architecture.

The computer operation instruction in an embodiment of the present disclosure may be a computer operation instruction based on the RISC-V instruction set architecture. Accordingly, the processor 402 may be designed based on the RISC-V instruction set. Specifically, a processor chip in the electronic device provided in an embodiment of the present disclosure may be a chip designed using the RISC-V instruction set. The chip can execute an executable code based on a configured instruction, thereby implementing the method for data processing in the above embodiments.

The memory 406 is configured to store the program 410. The memory 406 may include a high-speed RAM memory, and may further include a non-volatile memory, e.g., at least one disk memory.

The program 410 may specifically be used for causing the processor 402 to implement the method for data processing in any one of the above embodiments.

Corresponding description of the corresponding steps and units of the method for data processing in any one of the above embodiments may be referred to for specific implementations of the steps in the program 410, which will not be repeated here. Those skilled in the art can clearly understand that, for convenience and simplicity of description, the description of corresponding processes in the above method embodiments may be referred to for specific operating processes of the above-described devices and modules, which will not be repeated here.

With the electronic device in an embodiment of the present disclosure, during processing of the analog signal obtained by sampling the to-be-measured signal based on the target parameter, at least a portion of the analog signal is canceled with a signal of a signal value indicated by the target parameter, so that the first digital quantity is determined based on the cancellation signal, and then the first digital signal is compensated based on the target compensation amount, to obtain the measured signal value of the analog signal, and acquire the to-be-measured signal. The target parameter and the target compensation amount can be updated based on the first digital quantity. Compared with determining the first digital quantity directly based on the analog signal, the processable signal range for the processing module is extended by setting the target parameter and the target compensation amount in the present disclosure, thereby minimizing the problem that the processing module fails to acquire valid data during acquisition of the to-be-measured signal because the to-be-measured signal is either too large or too small, improving the efficiency of the measured signal value of the analog signal obtained by acquisition of the to-be-measured signal, and then improving the efficiency of signal acquisition.

Computer storage medium

The present disclosure further provides a computer-readable storage medium storing an instruction for causing a machine to implement the method for data processing as described herein. Specifically, a system or apparatus equipped with a storage medium may be provided, wherein the storage medium stores a software program code for implementing the functions of any one embodiment among the above embodiments and makes a computer (or CPU or MPU) of the system or the apparatus read and execute the program code stored in the storage medium.

In this case, the program code read from the storage medium itself can implement the functions of any one embodiment among the above embodiments, and thus the program code and the storage medium storing the program code constitute a part of the present disclosure.

Embodiments of storage mediums for providing the program code include a floppy disk, a hard disk, a magneto-optical disk, an optical disk (e.g., CD-ROM, CD-R, CD-RW, DVD-ROM, DVD-RAM, DVD-RW, or DVD+RW), a magnetic tape, a non-volatile memory card, and a ROM. Optionally, the program code may be downloaded from a server computer through a communication network.

Computer program product

An embodiment of the present disclosure further provides a computer program product, comprising a computer instruction, wherein the computer instruction instructs a computing device to implement the corresponding operations in any one of the above method embodiments.

It should be noted that user-related information (including but not limited to user device information, user personal information, etc.) and data (including but not limited to sample data for model training, data for analysis, stored data, displayed data, etc.) involved in the embodiments of the present disclosure are all information and data that are authorized by a user or fully authorized by all parties. Relevant data needs to be acquired, used, and processed following relevant laws, regulations, and standards of relevant countries and regions, and be provided with a corresponding operation portal for users to choose or refuse authorization.

It should be noted that, depending on the implementation requirements, the components/steps described in the embodiments of the present disclosure may be split into more components/steps, or two or more components/steps or partial operations of the components/steps may be combined into novel components/steps to achieve the goal of the embodiments of the present disclosure.

The above method according to the embodiments of the present disclosure may be implemented in hardware or firmware, or be implemented as software or a computer code storable in a recording medium (such as a CD ROM, a RAM, a floppy disk, a hard disk, or a magneto-optical disk), or be implemented as a computer code that is downloaded from a network, is originally stored in a remote recording medium or a non-transitory machine-readable medium, and will be stored in a local recording medium, such that the method described herein may be processed by such software stored on a recording medium using a universal computer, a dedicated processor, or programmable or dedicated hardware (such as an ASIC or FPGA). It is understandable that a computer, a processor, a microprocessor controller, or programmable hardware includes a storage component (e.g., a RAM, a ROM, or a flash memory) that can store or receive software or a computer code. The method described herein is implemented when the software or the computer code is accessed and executed by the computer, the processor, or the hardware. Further, when a universal computer accesses the code for implementing the method shown herein, the execution of the code converts the universal computer to a dedicated computer configured to implement the method shown herein.

It should be noted that user-related information (including but not limited to user device information, user personal information, etc.) and data (including but not limited to sample data for model training, data for analysis, stored data, displayed data, etc.) involved in the embodiments of the present disclosure are all information and data that are authorized by a user or fully authorized by all parties. Relevant data needs to be acquired, used, and processed following relevant laws, regulations, and standards of relevant countries and regions, and be provided with a corresponding operation portal for users to choose or refuse authorization.

As will be appreciated by those of ordinary skills in the art, the various example units and method steps described in combination with the embodiments disclosed herein can be implemented by electronic hardware, or a combination of computer software and electronic hardware. Whether these functions are executed by hardware or software depends on particular applications and design constraints of the technical solutions. Those skilled in the art may implement the described functions for specific applications using different methods, but such implementation should not be considered as falling beyond the scope of the embodiments of the present disclosure.

The above embodiments are only used to illustrate the embodiments of the present disclosure and are not intended to limit the embodiments of the present disclosure. Those of ordinary skills in the relevant technical field may further make various alterations and modifications without departing from the spirit and scope of the embodiments of the present disclosure. Therefore, all equivalent technical solutions are also encompassed within the scope of the embodiments of the present disclosure, and the scope of patent protection of the embodiments of the present disclosure should be defined by the claims.

**Claims**

1. A data processing method applied to a processing module, comprising:

   processing an analog signal obtained by sampling a to-be-measured signal based on a target parameter to obtain a first digital quantity, wherein the target parameter is used to indicate a signal value of a signal for cancelling at least a portion of the analog signal;
   compensating the first digital quantity based on a target compensation amount to obtain a measured signal value of the analog signal;

updating, when the first digital quantity satisfies a parameter update condition, the target parameter based on the first digital quantity to obtain an updated target parameter; and

updating the target compensation amount based on the updated target parameter to obtain an updated target compensation amount.

2. The method according to claim 1, wherein the updating the target compensation amount based on the updated target parameter to obtain the updated target compensation amount comprises:

processing the analog signal obtained by sampling the to-be-measured signal based on the updated target parameter to obtain a second digital quantity; and

updating the target compensation amount based on a difference between the first digital quantity and the second digital quantity to obtain the updated target compensation amount.

3. The method according to claim 2, wherein the updating the target compensation amount based on the difference between the first digital quantity and the second digital quantity to obtain the updated target compensation amount comprises:

computing the updated target compensation amount using a formula of:

$$dre\_code1 = H1(Rightshift(PpgMixData-PpgDreData, scale)+dre\_code0);$$

wherein dre_code1 is the updated target compensation amount, H1() is a first anti-overflow function, Rightshift(PpgMixData-PpgDreData, scale) is a value obtained by right shifting a difference between PpgMixData and PpgDreData by scale bits, PpgMixData is the first digital quantity in a binary form, PpgDreData is the second digital quantity in a binary form, scale is a number of right shifted bits, and dre_code0 is the target compensation amount.

4. The method according to claim 3, wherein the compensating the first digital quantity based on the target compensation amount to obtain the measured signal value of the analog signal comprises:

computing the measured signal value of the analog signal using a formula of:

$$PPG = H2(Rightshift(PpgMixData, scale)+dre\_code0);$$

wherein PPG is the measured signal value of the analog signal, H2() is a second anti-overflow function, and Rightshift(PpgMixData, scale) is a value obtained by right shifting PpgMixData by scale bits.

5. The method according to claim 1, wherein the analog signal is a current signal; and the processing the analog signal obtained by sampling the to-be-measured signal based on the target parameter to obtain the first digital quantity comprises:

outputting, via a signal output unit, a signal with a signal value indicated by the target parameter for cancelling the at least a portion of the analog signal to obtain a canceled analog signal;

converting the canceled analog signal via a trans-impedance amplifier included in the processing module into a voltage signal; and

converting the voltage signal via an analog-to-digital converter included in the processing module into the first digital quantity.

6. The method according to claim 5, wherein the updating the target compensation amount based on the updated target parameter to obtain the updated target compensation amount comprises:

determining the updated target compensation amount based on a signal value indicated by the updated target parameter, a gain resistance value of the trans-impedance amplifier, and an analog-to-digital conversion rule of the analog-to-digital converter.

7. The method according to claim 5, wherein the updating the target parameter based on the first digital quantity to obtain the updated target parameter comprises:

computing the updated target parameter using a formula of:

dc_cancel_code1 = H3((Idc_cancel0 + PpgMixData * A * 1000000/(TIA * D)) * B/C + dc_can cel_code0);

wherein H3() is a third anti-overflow function, dc_cancel_code1 is the updated target parameter, Idc_cancel0 is a signal value indicated by the target parameter, Idc_cancel0 is in a unit of microamperes, PpgMixData is the first digital quantity, A is a scale factor of the analog-to-digital converter, TIA is a gain resistance value of the trans-impedance amplifier, the TIA is in a unit of ohms, D is a maximum value outputted from the analog-to-digital converter, B is a maximum storage value at a storage bit used to store the target parameter, C is a maximum signal value of an output signal from the signal output unit, and dc_cancel_code0 is the target parameter.

8.  The method according to claim 1, wherein the processing module is configured to periodically sample the to-be-measured signal; and the method further comprises:
    determining that the first digital quantity satisfies the parameter update condition in response to the first digital quantity being a digital quantity corresponding to an analog signal obtained by an i-th sampling of the to-be-measured signal and a digital quantity corresponding to an analog signal obtained by each sampling among an (i-cnt+1)-th sampling to the i-th sampling of the to-be-measured signal being greater than or equal to a first threshold or being less than or equal to a second threshold, and otherwise determining that the first digital quantity fails to satisfy the parameter update condition, wherein each of the i and the cnt is a positive integer, the cnt is less than or equal to i, and the second threshold is greater than the first threshold.

9.  The method according to claim 1, wherein the processing module is configured to periodically sample the to-be-measured signal; and the method further comprises:
    stopping obtaining the measured signal value of the analog signal, in response to the first digital quantity being less than or equal to a lowest saturation threshold or being greater than or equal to a highest saturation threshold after obtaining the first digital quantity, until obtaining a first digital quantity less than the highest saturation threshold and greater than the lowest saturation threshold.

10. The method according to claim 1, wherein the compensating the first digital quantity based on the target compensation amount, to obtain the measured signal value of the analog signal is implemented by a range extension unit included in the processing module; and the method further comprises:
    resetting the range extension unit when the range extension unit is abnormal.

11. An electronic device, comprising: a processor, a memory, a communications interface, and a communication bus, wherein the processor, the memory, and the communications interface are configured to complete communication with each other through the communication bus; and
    the memory is configured to store at least one executable instruction, wherein the executable instruction causes the processor to implement corresponding operations of the data processing method according to any one of claims 1-10.

12. A computer storage medium, storing a computer program thereon, wherein the program, when executed by a processor, implements the data processing method according to any one of claims 1-10.

13. A computer program product, comprising a computer instruction, wherein the computer instruction instructs a computing device to implement the data processing method according to any one of claims 1-10.

Processing an analog signal obtained by sampling a to-be-measured signal based on a target parameter to obtain a first digital quantity ⟋101

Compensating the first digital quantity based on a target compensation amount to obtain a measured signal value of the analog signal ⟋102

Updating, when the first digital quantity satisfies a parameter update condition, the target parameter based on the first digital quantity to obtain an updated target parameter ⟋103

Updating the target compensation amount based on the updated target parameter to obtain an updated target compensation amount ⟋104

**FIG. 1**

THR_H

THR_L

Corresponding broken line of a compensated measured signal value

Compensating based on the updated target compensation amount

Corresponding broken line of the uncompensated first digital value

Target parameter update

**FIG. 2**

Preprocessing module 300

Signal processing unit
301

Value compensation
unit 302

First update unit 303

Second update unit
304

**FIG. 3**

Electronic device

Memory
406

Program
410

Processing unit
402

Bus
408

Communications
interface 404

**FIG. 4**

Europäisches
Patentamt
European
Patent Office
Office européen
des brevets

# EUROPEAN SEARCH REPORT

**Application Number**

EP 25 20 1668

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/037901 A1 (TRATTLER PETER [AT] ET AL) 6 February 2020 (2020-02-06) <br> * paragraph [0004] - paragraph [0010] * <br> * paragraph [0045] * <br> * paragraph [0050] - paragraph [0055] * <br> * paragraph [0104] - paragraph [0133] * <br> * figures 1-5 * | 1-13 | INV. <br> A61B5/024 <br> A61B5/00 |
| A | US 2013/120761 A1 (DYER KENNETH C [US] ET AL) 16 May 2013 (2013-05-16) <br> * paragraph [0019] * <br> * paragraph [0035] * <br> * figure 2D * | 1-13 | |
| A | US 2023/030688 A1 (NI JINHUA [CN] ET AL) 2 February 2023 (2023-02-02) <br> * paragraph [0025] * <br> * paragraph [0081] - paragraph [0095] * <br> * figures 4,5 * | 1-13 | |

**TECHNICAL FIELDS SEARCHED (IPC)**

A61B

The present search report has been drawn up for all claims

| Place of search | Date of completion of the search | Examiner |
|---|---|---|
| Berlin | 2 February 2026 | Völlinger, Martin |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.......................................................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 25 20 1668

This annex lists the patent family members relating to the patent documents cited in the above-mentioned European search report.
The members are as contained in the European Patent Office EDP file on
The European Patent Office is in no way liable for these particulars which are merely given for the purpose of information.

02-02-2026

| Patent document cited in search report | | | Publication date | Patent family member(s) | | Publication date |
|---|---|---|---|---|---|---|
| US 2020037901 | A1 | | 06-02-2020 | CN | 107920787 A | 17-04-2018 |
| | | | | EP | 3135196 A1 | 01-03-2017 |
| | | | | US | 2020037901 A1 | 06-02-2020 |
| | | | | WO | 2017032664 A1 | 02-03-2017 |
| US 2013120761 | A1 | | 16-05-2013 | CN | 103105612 A | 15-05-2013 |
| | | | | KR | 20130052486 A | 22-05-2013 |
| | | | | TW | 201320597 A | 16-05-2013 |
| | | | | US | 2013120761 A1 | 16-05-2013 |
| US 2023030688 | A1 | | 02-02-2023 | NONE | | |

EPO FORM P0459

For more details about this annex : see Official Journal of the European Patent Office, No. 12/82